(19) Europäisches Patentamt
European Patent Office
Office européen des brevets

(11) **EP 4 400 206 A1**

(12) **EUROPEAN PATENT APPLICATION**

(43) Date of publication:
**17.07.2024 Bulletin 2024/29**

(51) International Patent Classification (IPC):
**B01F 23/233** (2022.01)   **B01F 27/86** (2022.01)
**B01F 27/902** (2022.01)

(21) Application number: **23382029.9**

(22) Date of filing: **16.01.2023**

(52) Cooperative Patent Classification (CPC):
**B01F 23/23362; B01F 27/86; B01F 27/902;**
B01F 2215/0431

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Universidad de Sevilla
41013 Sevilla (ES)**

(72) Inventor: **DÁVILA MARTÍN, Javier
41092 Sevilla (ES)**

(74) Representative: **Hoffmann Eitle
Hoffmann Eitle S.L.U.
Paseo de la Castellana 140, 3a planta
Edificio LIMA
28046 Madrid (ES)**

(54) **AGITATED TANK WITH IMPROVED HOMOGENEITY BUBBLE DISTRIBUTION AND METHOD THEREOF**

(57)     The present invention refers to an agitator tank for the homogeneous distribution of bubbles in a liquid. The agitator tank comprises a liquid container 100, a gas injection system 120, at least one impeller 130, 140 and at least one baffle 150. The liquid container comprises a first and a second end defining the axial ends of the container and one or more sidewalls extending between the axial ends. The gas injection system is located proximal to the first end of the container. The at least one impeller is rotatable about at least one axis of rotation and comprises one or more blades extending to a maximum distance R from the axis of rotation of the corresponding impeller. The at least one baffle is axially displaced from the at least one impeller and fixed relative to the container at an orientation for reducing azimuthal flow generated by the at least one impeller. One or more impellers of the at least one impeller are shaped and oriented to induce axial flow of the liquid towards the gas injection system and the minimum distance of the at least one baffle to at least one axis of rotation is at most 1.2 R of any of the at least one impeller. The present invention also refers to a method thereof.

Fig. 1

**Description**

**Technical field of the invention**

The present invention relates to the field of the bioreactor devices, particularly to a bioreactor that can achieve a homogeneous distribution of bubbles with a low rotational speed and to a method of generating such distribution with low rotational speed.

**Background of the invention**

Different agitation and aeration systems for agitated tanks are known in the state of the art, incorporating agitators with different geometries. Said systems allow obtaining an adequate level of mixing with very high energy consumption, due to the fact that they must operate at a high rotational speed of the agitator or because the torque necessary to maintain said rotational speed is very high. Examples of agitation systems are summarised in the following patent documents.

US2019168177A1 describes a bioreactor for mammalian cell culture based on an axial-type agitator combined with an element with radial geometry outside the axial impellers that rotate in the part closest to the wall of the reactor.

KR1020160039907A describes an agitation system made up of discontinuous vertical baffles close to the side wall of the vessel.

US2019078045 A1 describes a compound agitation system with at least one upper impeller and one lower impeller, where the upper impeller is of the axial type.

WO2019126660 A1 describes a stirred reactor for the cultivation of microorganisms and cells in which the impeller that produces the circulation of fluids has pores on its surface to inject gas into the reactor.

The ability to achieve a homogeneous mixture with the described agitation systems is worse in some cases because several fluid recirculation cells are formed, which makes it difficult to achieve a complete mixture in a short time. This situation is typical of radial impellers such as the Rushton type.

In other agitation systems described, the high azimuthal velocity component of the fluid around the axis of the agitator induced by the rotation of the impellers makes it difficult to distribute the gas bubbles throughout the volume of the agitated tank. Some systems have vertical baffles close to the side walls of the vessel, but this system fails to efficiently reduce the azimuthal velocity component.

There is therefore a need for an agitation system for agitated tanks that can provide a homogeneous distribution of bubbles in a liquid with a low rotational speed of the agitator and with a reduced azimuthal velocity component.

**Summary of the invention**

A first aspect of the invention refers to an agitator tank for the homogeneous distribution of bubbles in a liquid. The agitator tank comprises a liquid container, a gas injection system, at least one impeller and at least one baffle. The liquid container comprises a first and a second end defining the axial ends of the container and one or more sidewalls extending between the axial ends. The gas injection system is located proximal to the first end of the container. The at least one impeller is rotatable about at least one axis of rotation and comprises one or more blades extending to a maximum distance R from the axis of rotation of the corresponding impeller. The at least one baffle is axially displaced from the at least one impeller and fixed relative to the container at an orientation for reducing azimuthal flow generated by the at least one impeller. One or more impellers of the at least one impeller are shaped and oriented to induce axial flow of the liquid towards the gas injection system and the minimum distance of the at least one baffle to at least one axis of rotation is at most 1.2 R of any of the at least one impeller.

In a preferred embodiment of the first aspect of the invention, a gap is provided between the at least one baffle and the one or more sidewalls.

In another preferred embodiment of the first aspect of the invention, the at least one impeller is a single impeller and the single impeller is shaped to induce at least axial flow of the liquid towards the gas injection system. Optionally, the single impeller is also shaped to induce radial flow from the axis of rotation of the single impeller to the one or more sidewalls. More preferably, one or more, and preferably all, of the blades of the single impeller comprise a surface having an average pitch angle between 10° and 60°, even more preferably, between 15° and 50°, wherein the average pitch angle is defined as the average angle formed between the chord line and the plane orthogonal to the axis of rotation of the impeller.

In another alternative preferred embodiment of the first aspect of the invention, the at least one impeller comprises a plurality of impellers axially displaced relative to one another. The plurality of impellers comprises a first impeller closest to the gas injection system and one or more second impellers farther from the gas injection system. The first impeller is shaped to induce at least radial flow of the liquid from the axis of rotation of the first impeller to the one or more sidewalls; and the one or more second impellers are shaped to induce at least axial flow of the liquid towards the first impeller.

In a more preferred embodiment, the one or more, and preferably all, of the blades of the first impeller comprise a surface having an average pitch angle between 50° and 90° and/or wherein one or more, and preferably all, of the blades of the one or more second impellers comprise a surface having and average pitch angle between 10° and 60°, preferably between 15° and 50°; wherein the average pitch angle is defined as the average angle formed between the chord line and the plane orthogonal to the axis of rotation of the impeller on which the blade is located. In another more preferred embodiment, the minimum distance between two adjoining impellers is less than 3R of any of said impellers. In another more preferred embodiment, the minimum diameter of the second impellers is greater than 0.3 times the square root of the vessel section perpendicular to the axis at the second impeller level, preferably greater than 0.45 times the square root of the vessel section perpendicular to the axis at the second impeller level.

In another preferred embodiment of the first aspect of the invention, the at least one baffle is folded or curved such that azimuthal flow of the liquid is converted into axial flow.

In another preferred embodiment of the first aspect of the invention, the minimum distance of the at least one baffle to at least one axis of rotation is less than R of any of the at least one impeller.

In another preferred embodiment of the first aspect of the invention, the gas injection system comprises injection orifices and wherein the average distance of the injection orifices to the first end is less than the distance R of the first impeller.

In another preferred embodiment of the first aspect of the invention, the gas injection system is a sparger comprising a plurality of orifices each located radially between the axis of rotation of the impeller closest to the first end of the container and the one or more sidewalls. Moreover, the average distance between the orifices of the sparger and the axis of rotation of the impeller closest to the first end of the container is larger than 0,7 R of the impeller closest to the first end of the liquid container.

In another preferred embodiment of the first aspect of the invention, the agitator tank comprises more than one impeller when the ratio between the height (H) of the liquid the agitator tank is configured to contain inside the liquid container and the mean of the diameter (<D>) of the one or more sidewalls is greater than 1.5 and more than 2 impellers if said ratio (H/<D>) is greater than 2. The liquid height (H) is the distance of such free liquid surface to the first end the agitated tank, and the mean of the diameter (<D>) is the mean of the square root of the surface of the vessel section perpendicular to the axis along the liquid container.

In another preferred embodiment of the first aspect of the invention, the at least one impeller comprises a hub with a radius greater than 0.2 R, preferably greater than 0.3R.

A second aspect of the invention refers to a method for homogeneously distributing bubbles in a liquid within an agitator tank, wherein the agitator tank is an agitator tank according to any of the embodiments of the first aspect of the invention. The method comprises the following steps:

a) generating at least an axial flow towards the first end of the agitator tank by means of one or more impellers of the at least one impeller above the gas injection system, preferably further generating a radial flow by means of the first impeller,
b) reducing the azimuthal flow generated by one or more of the impellers by the at least one baffle, and
c) injecting the gas into the fluid by means of the gas injection system proximal to the first end the container.

**Brief description of the drawings**

To enable better understanding of the present disclosure, and to show how the same may be carried into effect, reference will now be made, by way of example only, to the accompanying schematic drawings, in which:

Fig. 1 shows a schematic side view of an agitated tank according to at least one of the embodiments of the invention.

Fig. 2A shows a schematic top view gas injection system according to at least one of the embodiments of the invention.

Fig. 2B shows a schematic top view of another gas injection system according to at least one of the embodiments of the invention.

Fig. 2C shows a schematic top view of another gas injection system according to at least one of the embodiments of the invention.

Fig. 3A shows a schematic side view of an agitator tank incorporating a baffle according to at least one of the embodiments of the invention.

Fig. 3B shows a schematic radial cross-sectional view of the baffle of Fig. 3A, taken along line A-A.

Fig. 4A shows a schematic side view of an impeller configured to induce a radial flow according to at least one of the embodiments of the invention.

Fig. 4B shows a schematic top view of the shown in Fig. 4A.

Fig. 5A shows a schematic lateral view of an impeller configured to induce axial flow according to at least one of the embodiments of the invention.

Fig. 5B shows a schematic top view of the impeller shown in Fig. 5A.

Fig. 6 shows a schematic lateral view of a blade according to at least one of the embodiments of the invention.

Fig. 7 shows a simulated fluid flow within an agitator tank according to at least one of the embodiments of the invention.

Fig. 8 shows the mass transfer coefficient comparison of an agitator tank according to at least one of the embodiments of the invention with a standard stirred tank.

Fig. 9 shows a schematic diagram of a method for obtaining a homogeneous bubble distribution according to at least one of the embodiments of the invention.

## Description of the invention

### Definitions

The term "radial type" when referring to an impeller is preferably understood as an impeller configured to generate more radial flow than axial flow.
The term "axial type" when referring to an impeller is preferably understood as an impeller configured to generate more axial flow than radial flow.
The term "mixed type" when referring to an impeller is preferably understood as an impeller configured to generate both a radial and axial type of flow.
The term "proximal" with respect to one of the container ends is preferably understood as the element being closer to such end than to the other end of the container, preferably in the quarter closer to such end of the container.
The term "pitch angle" when referring to an impeller is preferably understood as the average angle formed between the chord line and the plane orthogonal to the axis of rotation of said impeller.
The term "sparger" is preferably understood as a gas injection system comprising a plurality of orifices configured to inject such gas through such orifices.

### Description

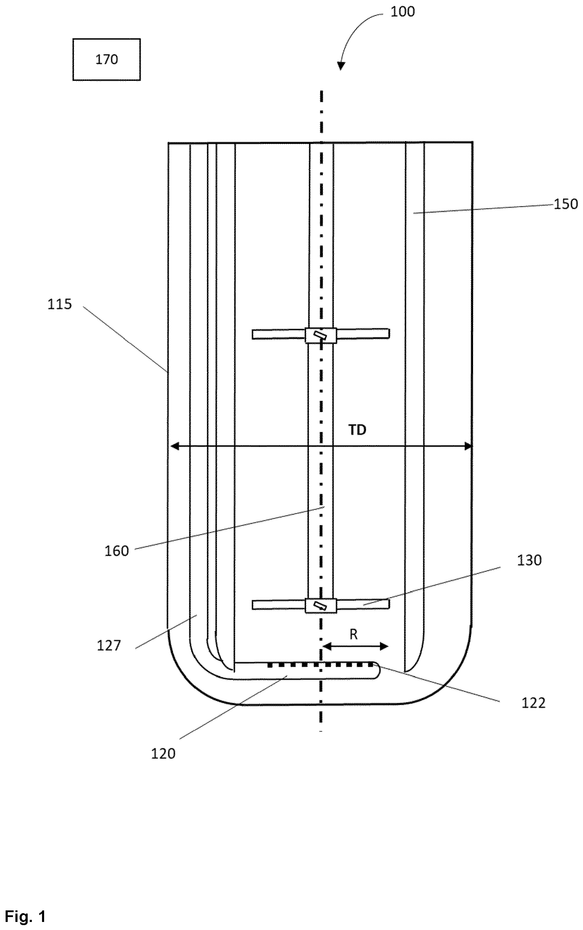

A first aspect of the invention refers to an agitated tank for the homogeneous distribution of bubbles in a liquid. It is noted that the agitator tank may be also known as agitated tank or stirred tank. As shown with respect to Fig. 1, the agitated tank comprises: a liquid container 100 comprising a first end 110 and a second end 112 defining the axial ends of the container 100 and one or more sidewalls 115 extending between the axial ends 110,112. The agitator tank also comprises a gas injection system 120, located proximal to the first end 110 of the container 100, and at least one impeller 130, 140 rotatable about at least one axis of rotation 160 (i.e. in embodiments where two or more impellers are provided, the axis of rotation of each impeller may be the same or different). The at least one impeller 130, 140 comprise one or more blades 130a, 130b, 130c, 140a, 140b, 140c extending to a maximum distance R from the axis of rotation 160 of the corresponding impeller 130, 140. At least one baffle 150 is provided axially displaced from the at least one impeller 130, 140, so that the baffle does not contact with any of the impellers 130, 140 when they rotate. The at least one baffle 150 is fixed relative to the container 100 at an orientation for reducing azimuthal flow generated by the at least one impeller 130, 140. It is also noted the at least one baffle 150 may reduce the azimuthal flow in different ways, for example it may simply reduce the flow by obstructing it or by redirecting it. One or more impellers of the at least one impeller 130, 140 are shaped and oriented to induce axial flow of the liquid towards the gas injection system 120. and the minimum distance D of the at least one baffle 150 to at least one axis of rotation 160 is at most 1.2 R of any of the at least one impeller.
It is noted that the liquid container 100 of Fig. 1 comprises two baffles 150, 150' but any number of baffles may be provided and in other embodiments only one baffle may be present. The one or more baffles may take any suitable shape and may be the same or different shapes as each other. Whilst the baffles 150 may extend the entire length of the container 100 between the first and second axial ends 110, 112, in other embodiments the baffles 150 may extend only part of the length. Also, the liquid container 100 comprises an optional second impeller 140, and other embodiments according to the present invention may not comprise a second impeller or there may be three or more impellers provided. Similarly, the first and second impellers 130, 140, respectively, have 4 blades each but in other embodiments they might comprise any other suitable number of blades. Moreover, the first end 110 of the liquid container 100 is shown as having a rounded shape, but this feature is optional, and other embodiments according to the first aspect of the invention may have any other suitable shape. Therefore, the different drawings must be taken for illustrative purposes only, and are not meant to limit the scope of the invention.

It is further noted that the second end 112 of the container 100 may be open or closed to the space external to the container 100. In some embodiments an openable or removable lid may be provided to fit to the second end 112 of the container 100 to close the container 100. The gas injection system 120 may take any suitable shape and comprise any number of orifices for ejecting gas.

In some embodiments and as shown in Fig. 1, the impellers may be mounted to a single rotatable shaft 170 configured to be driven by a motor, or they may be mounted to separate shafts. The separate shafts may have the same or different axes of rotation. The axes of rotation may be displaced and/or tilted with respect to one another. One or more of the axes of rotation may be centred with respect to the sidewalls 115 of the container 100 or off-centre with respect to the sidewalls 115.

The internal shape of the liquid container 100 may take several shapes. In some embodiments, for example, the liquid container 100 is a cylindrical shape with a defined base diameter and height, whilst in other embodiments it is a cube or a rectangular cuboid. In the case of a cylinder, the first and second ends 110, 112 may be defined by two circular planes on opposing faces. It is noted that oval, irregular or oblique versions of a cylinder may also be defined, in which case the two planes in opposing faces of the container may take oval or irregular shapes. Externally, the liquid container may take the same shape or a different shape as the internal shape of the liquid container. As the first and second ends define the axial ends of the container, the axis of rotation 160 of any of the impellers may be any axis which passes through both first and second ends 110, 112 of the liquid container or any other suitable axis which allows the one or more impellers to induce the fluid flow of the invention. Preferably, the axis of rotation 160 of any of the impellers 130, 140 are essentially centred in the first and second ends 110, 112 of the container.

As shown in Fig. 1 in some embodiments the gas injection system 120 is connectable to a gas source 170 through a gas conduit 127 configured to fluidly connect the gas from the gas source to the gas injection system 120. It is noted that the gas source may be a gas tank or reservoir, a gas generator like a compressor or any other source of gases that can provide gas to the gas injection system 120 in a controlled way. The gas may be any suitable gas required for whichever reaction the agitator tank 100 is configured to perform. It is also noted that the gas conduit 127 may take several forms and be provided in different ways to the liquid container and that the design showed in Fig. 1 must be only taken as a representation of such form. For example, the conduit may be provided internally to the container 100, may extend along or inside the sidewall 115 or may extend along the sidewall 115 external to the container 100.

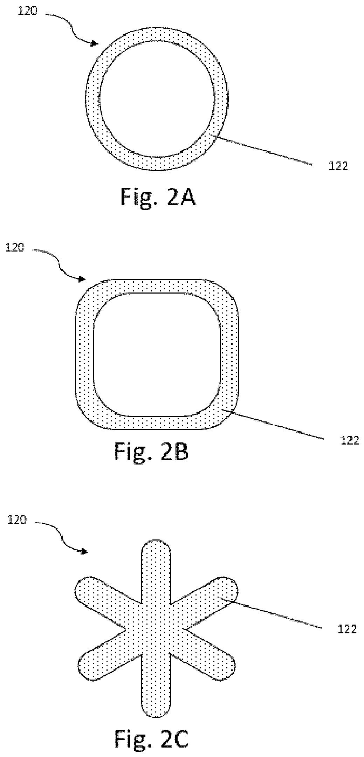

The gas injection system 120 may take any suitable shape and may comprise any number of orifices for expelling the gas. As shown in Figs. 2A- 2C the gas injection system 120 is a device configured to introduce bubbles of a gas into a liquid. The gas injection system 120 may take several shapes. For example, the gas injection system 120 may take a circular or toroidal shape as shown in Fig 2A or more complex shapes such as a squared toroid as shown with respect to Fig. 2B or such as a star-shaped sparger comprising a plurality of cylindrical gas injectors arranged in a radial disposition as shown with respect to Fig. 2C. It may also take the shape of a plurality of cylindrical gas injectors disposed parallel to each other. It is noted that the person skilled may implement different possible configurations of the gas injection system as known in the art.

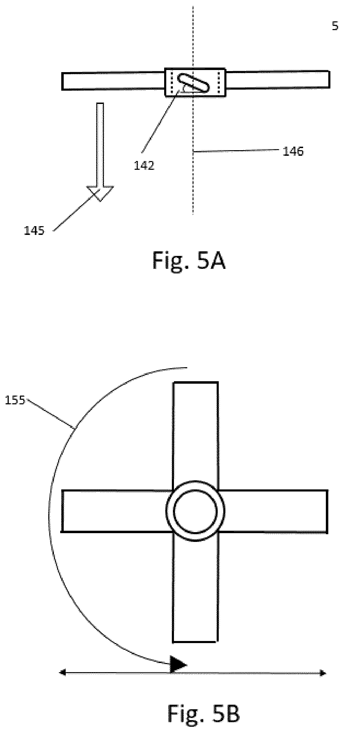

The at least one impeller 130, 140 comprises at least one blade. It preferably comprises two or more blades. The at least one has a defined first axis of rotation 136. One or more impellers of the at least one impeller 130, 140 are shaped to generate an axial flow 145 relative to the axis of rotation of the one or more impellers 130, 140 as shown with respect to Fig. 5A and 5B.

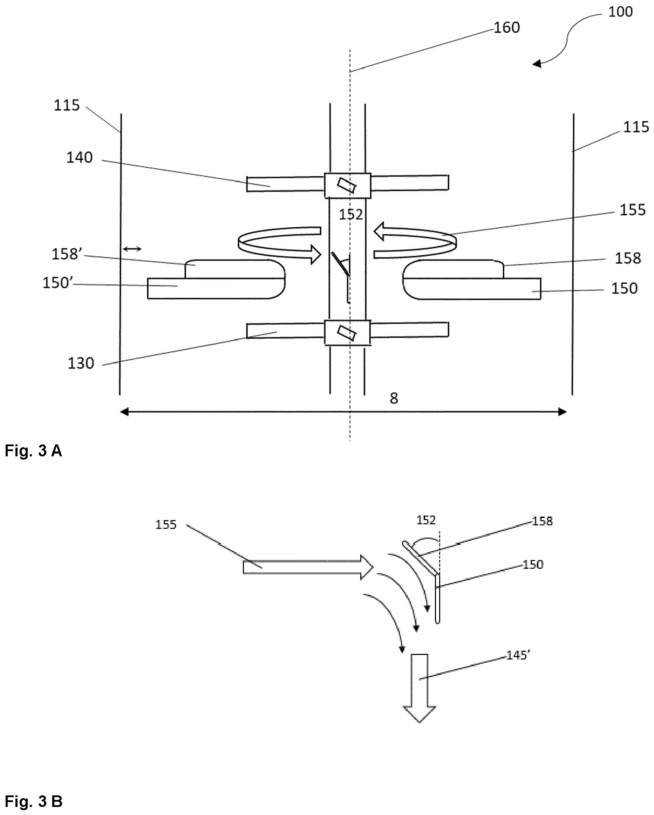

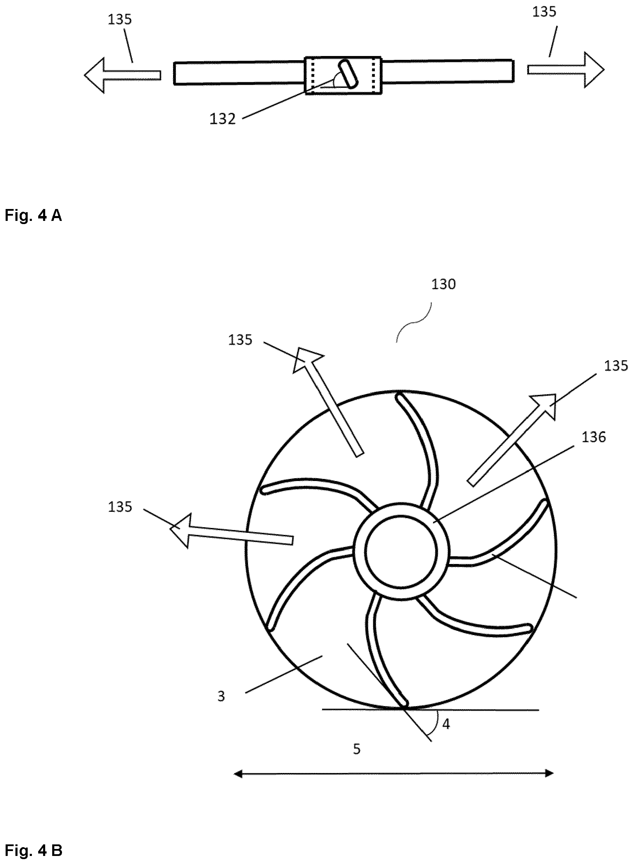

Fig. 3A, shows a schematic side view of an agitator tank incorporating a baffle 150 according to at least one of the embodiments, wherein the fluid flow of the liquid when the agitator tank is in use is shown. It is noted that the container 100 of the Fig. 3 comprises an optional second baffle 150' and a second impeller 130. The impeller 140 generates an axial flow 145 and an azimuthal flow 155 as a side effect from the impeller 140 rotation. The azimuthal flow 155 is defined as the flow around the axis of rotation with an azimuthal direction of such impeller as shown in Fig. 4B and 4B. The azimuthal flow is counterproductive for an agitated tanks where bubble homogeneity is desired, as the bubbles coalescence near the rotation axis due to the centripetal force generated. Therefore, the azimuthal flow 155 is not desired and its reduction enhances the bubble homogeneity distribution. The at least one baffle 150, is configured to reduce the azimuthal flow 155. It is noted that in those embodiments wherein the second impeller 140 is not present, the at least one baffle 150 would be configured to reduce the azimuthal flow 155 from the impeller. It is noted that the second planes 158, 158' are optional.

A baffle 150 at least partially perpendicular to the azimuthal flow 155 of an impeller, reduces the azimuthal flow 155 generated by such impeller. The person skilled in the art will note that there are several ways of setting a baffle such that it reduces the azimuthal flow. As shown in Example 2, the presence of a baffle significantly reduces the azimuthal flow of the fluid caused by rotation of the impellers, while in Example 3, it is shown that the presence of the baffle increases the mass transfer due to a larger homogeneity of the bubble distribution in the agitated tank. In some embodiments, as shown in Fig 3A, the baffle is further configured to transform the azimuthal flow 155 into an axial flow 145'. This is further seen with respect to Fig. 3B, which shows a schematic cylindrical cross-sectional view of the baffle of Fig. 3A, taken along line A-A as a cross-section generated by a cylindrical surface concentric with the axis of rotation of the at least one impeller would do.

It is noted that the term partially perpendicular is understood as having a surface with an angle comprising a perpendicular component to the azimuthal flow 155. Thus, any baffle with a surface not parallel to the azimuthal flow, with be partially perpendicular. It is also noted that, by design, any surface not parallel to a flow will reduce such flow by redirecting it, thus a surface which is partially perpendicular to the azimuthal flow 155 reduces the azimuthal flow 155.

The baffle 150 may be fixed relative to the liquid container 100 in different ways. The baffle may be fixed to the sidewalls 115 of the liquid container 100, and/or may be fixed to the first and/or second ends 110, 112 of the liquid container 100. It is noted that the baffle 150 may be directly fixed or fixed through an arm (not shown). The baffle 150 may be integral with the container 100 or may be attached to the container by fixing means, such as a screw, clamp, or by welding or adhesive. In any of the embodiments with more than one baffle 150 as shown in Fig. 1, the baffles may be fixed relative to the liquid container 100 in different positions with respect to the axis of rotations of one of the impellers 130, 140, and/or the first and second ends of the liquid container 110, 112. Each of the one or more baffles may be configured to reduce the azimuthal flow generated by one or more impellers. Also, the more than one baffle may have different shapes and sizes between them, and their shape and size may be determined by the by the impellers whose azimuthal flow 155 they are configured to reduce. For example, a baffle may have a different shape and/or size if it's configured to reduce the azimuthal flow of an impeller with bigger blades compared to another baffle configured to reduce the azimuthal flow of an impeller with smaller blades. This may be the case for any parameter related to the impeller, such as the flow it generates, position in the agitator tank, size of the blades, etc. The person skilled in the art may foresee that the impellers can be characterised by many other parameters and the corresponding baffle may be designed according to these parameters.

In a preferred embodiment, the minimum distance of the at least one baffle to at least one axis of rotation is less than R of any of the at least one impeller; such that the baffle 150 overlaps with at least one blade of the one or more impellers 130, 140 (i.e. when viewed down the axis of rotation of one of the impellers). It is noted that in Fig. 1, the baffle 150, comprises a protruding section 151 with a distance to the at least one axis of rotation 160 less than R, so that the baffle overlaps with at least one blade of the at least one impeller 130.

In another preferred embodiment, a gap 154 is provided between the at least one baffle 150 and the one or more sidewalls 115. Therefore, the at least one baffle 150 mainly reduces the azimuthal flow where this is maximum, i.e. at an intermediate distance between the at least one axis of rotation 160 and the sidewall 115 at the distance R, which corresponds to the extension of the blades of the at least one impeller 130. As shown in Fig. 3A the at least one baffle 150 leaves a gap 154 at the sidewall 150. Advantageously this makes a more efficient use of the baffle 150 surface, by concentrating its azimuthal reduction where the azimuthal flow is maximum.

In a preferred embodiment, the gas injection system 120 comprises a plurality of orifices 122 each located radially between the axis of rotation 160 of the impeller closest to the first end of the container and the one or more sidewalls 115. The gas injection system 120 may be a sparger such as a ring sparger. The orifices 122 are the injection locations of the gas into the liquid container 100, therefore providing bubble generation points. The radial location of such orifices 122 ensures that the gas is injected further from the at least one axis of rotation 160. This is preferred because any azimuthal flow generated in the flow tends to drive gas bubbles towards the axis of rotation, so a more homogeneous distribution of bubbles is achieved when the gas bubbles are injected away from the axis of rotation. The sparger 120 comprises a set of orifices 122 through which the bubbles are introduced into the liquid. It is noted that these set of orifices 122 may be distributed in different ways through the surface of the sparger. These may be evenly distributed, or unevenly distributed through the surface of the sparger 120. The person skilled in the art will note that different distributions can be achieved with different spargers to achieve a desired gas injection. For example, the set of orifices 122 may be distributed unevenly, by considering the distance of the gas injection system 120 to the sidewalls of the liquid container 100, and/or may be distributed by considering the distance of the gas injection system 120 to the first and second ends 110, 112 of the liquid container 100. For example, the orifices 122 may be more densely distributed, the closer they are to the sidewalls of the liquid container and the closer to the first end 110 of the liquid container 100. The set of orifices 122 may be a set of equally-sized orifices or orifices of different sizes, that may also be evenly or unevenly distributed through the surface the sparger 120. It is also noted that the sparger 120 may be a dedicated system as shown in Fig. 1 or may be defined within the liquid container, for example, by the definition of a set of orifices on the first end 110 through which gas is injected. Thus, the term sparger may be understood as any gas injection system comprising a plurality of orifices for such gas injection.

More preferably, the sparger 120 of the preferred embodiment comprises orifices 122 homogeneously distributed orifices 122. As shown in Fig. 2A-2C may comprise the orifices for gas injection 122 into the liquid homogeneously distributed on its surface. Advantageously, the homogeneous disposition of the orifices on the surface of the sparger further increases the homogeneity of the bubbles in the container.

In a more preferred embodiment, wherein the average distance between the orifices of the sparger 120 and the axis of rotation 160 of the impeller closest to the first end 110 of the liquid container 100 is larger than 0,7 R of the impeller closest to the first end 110 of the liquid container 100. Even more preferably, at least 75% of the gas flow rate is injected at a distance greater than 0.7R from the axis of rotation 160 of the impeller closest to the first end 110. Advantageously, this avoids the gas bubbles to not be attracted towards the axis, which would reduce the homogeneity of the bubbles in the container.

In another preferred embodiment of any of the previous embodiments, the at least one impeller 130 is a single impeller and the single impeller is shaped to induce at least axial flow 145 of the liquid towards the gas injection system 120, optionally wherein the single impeller 135 is also shaped to induce radial flow 135 from the axis of rotation 136 of the single impeller 135 to the one or more sidewalls 115. When only one impeller 130 is provided, it is shaped to induce at least axial flow 145 of the liquid towards the gas injection system 120. This ensures that the liquid flow generated is circulatory toward the gas injection system 120 close to the axis of rotation 136 of the single impeller 135 and from the gas injection system 120 close to the one or more sidewalls. As the bubbles generally have a tendency to move upwards towards the surface of the liquid, upward flow of the liquid is allowed in the outer radial part of the container 100. The downward flow generated by the single impeller at a radially inner location of the container 110, combined with the natural upward flow of the bubbles radially away from the single impeller, means that circulatory flow of the fluid is induced in the container 100. The one or more baffles 150 inhibit azimuthal flow from being generated so that the radial distribution of the bubbles is homogeneous.

Therefore, in use the flow generated by the single impeller 145 interacts with the gas provided by the gas injection system 120 and by reaching the first end 110 of the container 100, it returns to the second end of the container 112 along a path close to the one or more sidewalls 115 (i.e. along a radially-outer path of the container 100). Optionally, the single impeller 130 is also shaped to induce radial flow 135 from the axis of rotation 136 of the single impeller 135 to the one or more sidewalls 115, which further induces outer radial flow from the axis of rotation 160 towards the one or more sidewalls 115 thereby encouraging the desired circulatory flow of the gas bubbles in the fluid.

As shown in Fig. 5A the single impeller 130 may have an pitch angle 142. The pitch angle 142 influences the flow 145 generated. A higher pitch angle 142 configures the single impeller 130 to have a more mixed type of flow. Generally speaking, the lower the angle, the more axial the flow 145 is (i.e. the generated flow has a lower radial component). In more preferred embodiment, the one or more, and preferably all, of the blades of the single impeller comprise a surface having an average pitch angle between 10° and 60°. The average pitch angle is defined as the average angle formed between the chord line and the plane orthogonal to the axis of rotation of the impeller. Thus, the average pitch angle $\langle\alpha\rangle$ can be expressed as:

$$\langle\alpha\rangle = \frac{\int_{r_{min}}^{r_{max}} \alpha(r)\, r\, dr}{\int_{r_{min}}^{r_{max}} r\, dr}$$

wherein, alfa(r) is the pitch angle of the section generated by a cylindrical surface of radius r around the axis of rotation of said impeller.

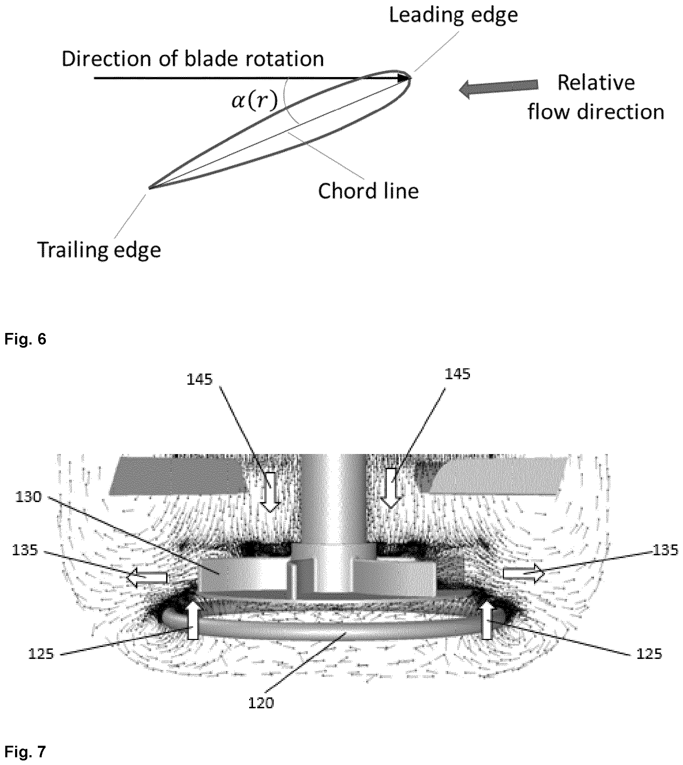

As shown with respect to Fig. 6, the chord line is defined as the line connecting the leading edge and the trailing edge of a blade for a determined cut equidistant to the axis, and the pitch angle at a certain radius r of the blade is defined as the angle formed between the chord line at such radius and the orthogonal plane to the axis of rotation of the impeller, this is the direction of rotation. By integrating this angle along the blade for all its radius $r_{min} < r < r_{max}$, and averaging the values weighted by the distance to the axis of rotation r, the average pitch angle $\langle\alpha\rangle$ can be computed.

More preferably, the average pitch angle of said surface is between 15° and 50°. Advantageously, this ensures that the flow 145 of the single impellers 130 has a large downward axial flow component and a low radial component. It is noted that each blade of the impeller may have a different average pitch angle 142, and that the pitch angle 142 of each blade may change arbitrarily over its radius, or they may be designed to change considering their distance to one of the ends of the container 110, 112 and/or according to their distance to the axis of rotation 160.

In an alternative preferred embodiment of any of the previous embodiments, as shown with respect to Fig. 1. the at least one impeller comprises a plurality of impellers axially displaced relative to one another, wherein the plurality of impellers comprise:

- a first impeller 130 closest to the gas injection system 120, the first impeller 130 shaped to induce at least radial flow 135 of the liquid from the axis of rotation of the first impeller 135 to the one or more sidewalls 115; and
- one or more second impellers 140 farther from the gas injection system 120, the one or more second impellers 140 shaped to induce at least axial flow 145 of the liquid towards the first impeller 130.

Therefore, the first impeller 130 is longitudinally displaced from the gas injection system 120 towards the second end 112 and positioned between the gas injection system 120 and the one or more second impellers 140. Advantageously, in use this agitator tank can produce a circulatory flow in a longer container, i.e. wherein the axial ends 110, 112 are further away. Only the first impeller 130 requires generating a radial flow that moves the axial flow from the one or more second impellers 140 towards the sidewalls, as it is the lower impeller.

The one or more second impellers 140 are longitudinally displaced towards the second end of the liquid container 112 and positioned between the first impeller 130 and the second end of the liquid container 112. Advantageously, in use this configures the one or more second impellers 140, which are the upper impellers to encourage the axial flow 145 so that a single circulatory loop along the length of the liquid container 100 is achieved.

The one or more second impellers 140 comprises at least one impeller. Each of the one or more second impellers 140 may comprise any number of blades. The one or more second impellers 140 define at least a second axis of rotation 146 which may be the same or different (translated or tilted) with respect to one another, and the same or translated/tilted with respect to the first impeller. It is noted that the one or more second impellers 140 may comprise one, two or more impellers (140, 140', 140"). In the embodiments wherein there is more than one second impellers, each second impeller may comprise different or equal number of blades. It is noted that as the first and the one or more second impellers first and second axis of rotation 136, 146, respectively, may be different between them. Each second impeller 130 comprises at least one blade. It preferably comprises two or more blades.

The first impeller 130 is shaped to generate a radial flow 135 relative to the axis of rotation of the first impeller 136 as shown with respect to Fig. 4A and 4B and the one or more second impellers 140 are shaped to generate an axial flow 145 relative to the axis of rotation of the one or more second impellers 146 as shown with respect to Fig. 5A. It is noted that the respective radial and axial flows 135, 145 may be exclusively axial and radial, respectively. In some other embodiments, the first impeller 130 and the one or more second impellers 140 may be configured to generate an essentially radial and axial flow respectively, wherein most of the flow is radial and axial respectively, but a small axial and radial flow is also generated, respectively. Also, the impellers may generate a mixed type of flow, with a similar amount of radial and axial flow. In a preferred embodiment of all embodiments where the tank comprises such a first impeller and one or more second impellers, the first impeller are configured to generate more radial flow than axial flow and the one or more second impellers generate more downward axial flow than radial flow.

In use the impellers 130, 140 intervene in the free path of the gas from the gas injection system 120 on its way to the fluid surface and the flow created by the impellers 135, 145 is recirculatory, defined by the radial flow 135 created by the first impeller 130 and the axial flow 145 towards the gas injection system 120 created by the one or more second impellers 140. As in use the first end of the container 110 is located at the bottom, the gas injection system 120 is located underneath all of the impellers 130, 140 of the liquid container 100. Therefore, the free path of the gas from the gas injection system 120 defines a free path via the impellers 130, 140. The combination of a first radial impeller 130 and one or more second axial impellers 140 above the first impeller 130 generates a circulatory flow that flows towards the gas injection system 120 closer to the at least one of the axis of rotations 136, 146, and in the opposite direction away from the gas injection system 120 and towards the surface of the liquid closer to the one or more sidewalls 115 of the liquid container 100. The first impeller 130 being of the radial type modifies the axial flow 145 generated by the one or more second impeller 140 so that the flow direction is driven from the axis of rotation of the first impeller 130 towards the one or more sidewalls 115. The one or more second impeller 140 are configured to generate a flow 145 towards the first impeller 130 and therefore toward the first end of the container 110, and the first impeller 130 is configured to generate a radially outward flow 135 from the axis 136, so that the circulatory flow according to the invention is generated.

As shown in Fig. 4A the impellers of the first impeller 130 may have an pitch angle 132. The pitch angle 132 influences on the flow 135 generated as a lower pitch angle configures the first impeller 130 to have a more mixed type of flow. The bigger the pitch angle 132, the more radial the flow 135. In a more preferred embodiment, the blades of the first impellers comprise a surface having an average pitch angle between 50° and 90° along at least part of the surfaces of the blades. Advantageously, this ensures that the flow 135 of the first impeller 130 is at least radial flow. It is noted that each of the blades may have a different average pitch angle 132, and that the pitch angle 132 of each blade may change through its surface according to the distance to one of the ends of the container 110, 112 or according to the distance to the axis of rotation 160.

As shown in Fig. 4B the blades of the first impeller 130 may have a curved surface, with a different exit angle 4 along the length of the blades. More preferably, the exit angle is smaller as the blade is further from the axis of rotation 136. Advantageously, this allows the radial flow 135 to be increased as the liquid is further from the rotation axis 136. Also, the blades of the first impeller 130 may comprise a surface 3 affixed to the blades in the closest most part to the gas injection system 120. This surface 3, prevents to the gas bubbles to be suctioned by the impeller 130 and therefore the azimuthal flow 135 generated contains less gas bubbles, as they can only escape at the ends of the impeller 130, closer to the sidewalls of the liquid container 100.

As shown in Fig. 5A the impellers of the one or more second impellers 140 may have an pitch angle 142. The pitch angle 142 influences on the flow 145 generated as a bigger pitch angle configures the one or more second impellers 140 to have a more mixed type of flow, the lower the angle, the more axial the flow 145 is. In another more preferred embodiment, the one or more, and preferably all, of the blades of the one or more second impellers comprise a surface having an average pitch angle between 10° and 60°. More preferably, the average pitch angle of said surface the one or more second impellers is between 15° and 45°. Advantageously, this ensures that the flow 145 of the one or more second impellers 140 is at least axial flow. It is noted that each of the blades may have a different average pitch angles 142, and that the pitch angle 142 of each blade may change through its surface according to the distance to one of the ends of the container 110, 112 or according to the distance to the axis of rotation 160. Moreover, in the embodiments wherein the agitated tank comprises more than one second impeller 140, 140', the pitch angle 142 may be different between each second impeller 140, 140'.

In a preferred embodiment of any of the previous embodiments, the at least one baffle 150 is folded or curved such that azimuthal flow 155 of the liquid is converted into axial flow 145'. As shown in reference to Fig. 3A and 3B the at least one baffle 150 may comprise a second plane 158 with a specific angle 152 with respect to a plane containing the axis of rotation 160 and/or it may define a curve anywhere between a plane perpendicular to the axis of rotation 160 and a plane parallel to the axis of rotation 160. Advantageously, this allows the baffle 150 to employ part of the undesired azimuthal flow 150 to generate the desired recirculatory flow, by converting part of the azimuthal flow 155 of the liquid into downward axial flow 145. The skilled person will understand that the fold or curve can be any suitable shape configured to convert azimuthal flow into downward axial flow. In some embodiments, as shown in Fig. 3B, a radially inward part of the baffle 150 comprises a folded or curved section as described, and a radially outer part of the baffle 150 is free from any such folds or curves so that azimuthal flow is converted into downward axial flow only at radially inward locations where the downward flow is desired. As shown in Fig. 3B, which shows a cross-sectional radial view of the baffle 150 along ling A-A, a baffle 150 comprising a second plane 158 with an angle 152 with respect to a plane containing the axis of rotation redirects the azimuthal flow 155 into an axial flow 145'.

In use as shown in Fig. 3A, the agitator tank when comprising the first impeller and the two or more second impellers is configured to generate an axial flow 145 with the at least one second impeller 140 that makes the liquid travel towards the first impeller 130. However, due to the rotation of the at least second impeller 140 around the axis 160, an azimuthal flow 155 is generated. To reduce the azimuthal flow, the baffles 150, 150' are provided, each comprising a second plane 158, 158' with an angle 152 with respect to a plane containing the axis of rotation, that together with the main plane of the baffle 150, 150', transforms the azimuthal flow 155 into an axial flow 145'. It is noted that the second planes 158, 158' are optional.

In a more preferred embodiment, the angle 152 of at least part of the baffle 150 forms an average angle of between 10° and 45° with the main plane of the baffle 150. Advantageously, this increases the azimuthal flow 155 reduction. By establishing an inclined angle with the axis of rotation of at least one impeller, the baffle increases the interaction with the azimuthal flow 155 more efficiently, further reducing it and advantageously generating an axial flow 145'. For example, as shown in Fig. 3B, the baffle comprises a section 158 that defines a second plane of the baffle 150. This section 158 is with an angle 152 compared to the main plane of the baffle 150, which in this case is the plane perpendicular to the azimuthal flow.

It is noted that the at least one baffle 150 is preferably at least partially perpendicular to the azimuthal flow 155 of a set of impellers in different ways. For example, the at least one baffle 150 may define a plane crossing the axis of rotation of one set of impellers that extends radially towards the axis of rotation and absolutely perpendicular to any generated azimuthal flow 155. In the embodiments, wherein the section 158 with an angle 152 is defined, this section 158 may also extend radially towards the axis of rotation.

The at least one baffle 150 may take different shapes and angles arbitrarily along its length. However, it also may be designed to take the different shapes and angles depending on the distance of a specific section of the baffle to the axis of rotation of the impeller whose azimuthal flow is configured to reduce, and/or to its distance to the first and second ends 110, 112 of the liquid container 100. It may also take different shapes in each face, with one parameters for the face facing the azimuthal flow and others for the other face not facing the azimuthal flow. Therefore, the sense of rotation of the impeller may be also considered. For example, the baffle 150 may have a longitudinally bigger extension closer to the axis of rotation and a longitudinally lower extension closer to the sidewall of the container, and/or it may have a bigger or smaller angle as it gets closer to one of the ends 110, 112 of the liquid container 100. It is noted that when more than one baffle 150 is present, the baffles 150 may be different between them. For example, they might have different shapes, number of sections 158, and angles 152 based on their distance to the impeller whose azimuthal flow 155 they reduce, distance to the ends 110, 112 of the liquid container 100, and/or distance to the axis of rotation 160 of the impeller, for example.

In another preferred embodiment, the gas injection system comprises injection orifices and the average distance of the injection orifices to the first end should is less than the distance R of the first impeller. Advantageously, this guarantees that a more homogeneous distribution of bubbles is achieved, because as mentioned above, any azimuthal flow generated in the flow tends to drive gas bubbles towards the axis of rotation.

In another preferred embodiment, the minimum distance between two adjoining impellers is less than 3R of any of said impellers. When two adjacent impellers are too far away from each other, there is a risk the flow generated by one does not reach the other one, therefore producing a volume wherein no flow or a local circulatory and undesired flow is generated. By setting the impellers 130, 140 at a minimum distance between each two adjacent ones of less than 3R of any of said impellers., it configures the agitated tank to reduce or eliminate the dead areas, i.e. areas without flow, or areas with local undesired circulatory flows between the impellers, as the flow generated by one set of impellers effectively reaches the other one, so that a unique flow is generated.

In another preferred embodiment, the minimum diameter of the second impeller 140 is greater than 0.3 times the square root of the vessel section perpendicular to the axis at the second impeller level. More preferably, the minimum diameter of the second impeller 140 is greater than 0.45 times the square root of the vessel section perpendicular to the axis at the second impeller level.

As the one or more second impellers 140 is of an axial type, the axial flux it generates directs the gas bubbles axially to the adjacent set of impellers 130 or 140. If the diameter of the one or more second impellers 140 is too low, the gas bubbles would not effectively be directed towards the adjacent set of impellers 130 or 140 and instead several local circulatory flows would be generated in the volume between the generated axial flow 145 and the flow generated in the opposite sense closer to the surface of the container 100 by the first impeller 130. Therefore advantageously, having a minimum diameter of the second impeller 140 greater than 0.3 times the diameter TD ensures the effective direction of the gas bubbles to the adjacent set of impellers 130 or 140.

In another preferred embodiment, the agitator tank comprises more than one impeller when the ratio between the height (H) of the liquid the agitator tank is configured to contain inside the liquid container and the mean diameter (D) of the one or more sidewalls is greater than 1.5, and more than two impellers than 2 if said ratio (H/D) is greater than 2. Advantageously, this ensured that when the agitator tank has an elongated shape, the number of impellers are accordingly settled, such that the flow between the first end 110 of the liquid container and the liquid surface is homogeneous.

The liquid height (H) is the distance of such free liquid surface to the first end the agitated tank. It is noted that although the agitator may not comprise the liquid, it is configured to stir and mix homogeneously the liquid with the air, and it is configured to work with a certain amount of liquid. Thus, it is understood that those embodiments wherein the container does not comprise the liquid, as long as it is configured to contain inside the liquid container a liquid with such height (H), such that the defined ratio is met, they are comprised within this preferred embodiment.

The mean diameter (D) is the square root of the surface of the vessel section perpendicular to the axis along the liquid container. The mean diameter can be expressed as:

$$\langle D \rangle = \frac{1}{H} \int_{z_{min}}^{z_{max}} D(z)\, dz$$

wherein D(z) is the diameter at an axial distance z from the first end of the agitated tank, defined as the square root of the surface of the vessel section perpendicular to the axis at an axial distance z from the first end of the agitated tank.

In another preferred embodiment, the at least one impeller comprises a hub with a radius greater than 0.2 R, preferably greater than 0.3R. Advantageously, this avoids that bubbles can accumulate near the axis, at 0.2R or closer, thus ensuring a greater homogeneity.

In order to further reduce the formation of dead volumes, i.e. volumes where liquid velocity is much smaller than the average velocity inside the stirred tank, typically less than 10% or where local recirculatory flows are generated, the shape of the ends of the liquid container 100 may be curved. For example, if the ends 110, 112 have straight angles when they contact the lateral surface of the container, local recirculatory flows may be generated. In a preferred embodiment, the first end of the container 110 has a rounded shape such that the interface between the first end 110 and the one or more sidewalls 115 is free from vertices to prevent the formation of dead flow areas. This generates a surface that allows the circulatory flow of the liquid to be formed closer to the first end 110 of the container 100. Advantageously, in use this reduces the formation of dead ends on the first end 110 of the container 100.

In another preferred embodiment, the agitator tank further comprises a rounded sidewall element at the height (H) of the liquid the agitator tank is configured to contain inside the liquid container 100, the rounded sidewall element configured to replicate the sidewall of the liquid container with a curved shape. Advantageously, it allows to adapt the sidewalls to be rounded at such height, therefore imitating the rounded ends 110, 112 reducing the formation of dead ends close to the second end 112 at the height (H) of the liquid the agitator tank is configured to contain. Therefore, this generates a surface that allows the circulatory flow of the liquid to be formed on the surface of the liquid. More preferably, the rounded sidewall element is configured to be displaceable longitudinally along the liquid container between the first end 110 and the second end 112. Advantageously, this allows to adapt the rounded sidewall element to different heights (H) of liquid the agitator tank may be configured to contain inside the liquid contain 100. Thus, prior to filling the liquid container 100, the user may set the rounded sidewall element to the height of the liquid it contains or will contain, therefore configuring the container to reduce local recirculatory flows on the surface of the liquid, close to the second end 112.

It is noted that the agitated tank, including any of the elements comprising the agitated tank may be comprised of different materials. In a preferred embodiment of any of the embodiments of the first aspect of the invention, the agitated tank comprises a material selected from any of the following materials: metals such as steel and/or thermoplastics.

A second aspect of the invention refers to a method for homogeneously distributing bubbles in a liquid within an agitator tank, wherein the agitator tank is any of the agitator tanks of the first aspect of the invention.

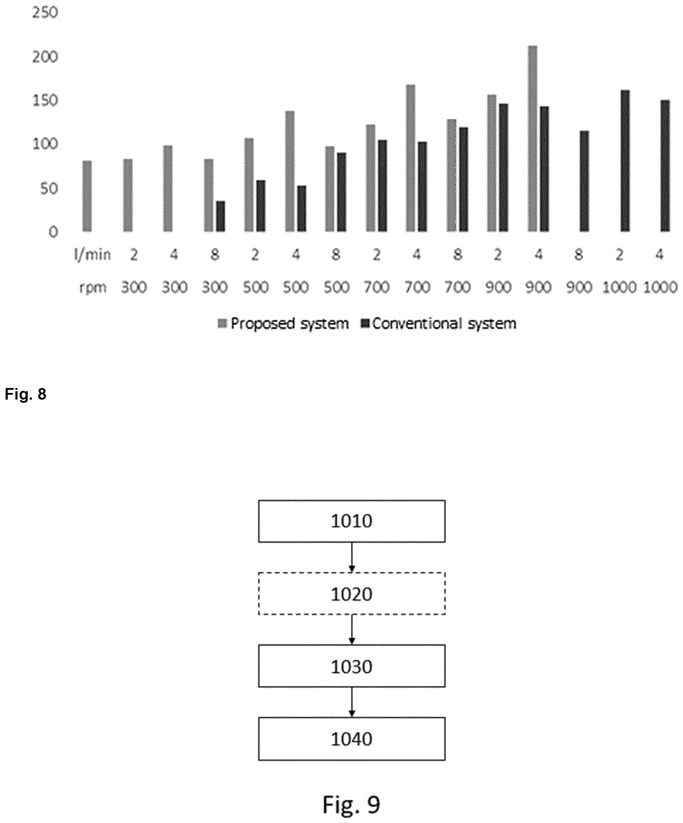

As shown with respect to Fig. 9, the method first comprises the steps 1010 of generating at least an axial flow towards the first end of the agitator tank by means of one or more impellers of the at least one impeller above the gas injection system near the bottom of the container. The axis of rotation is defined by a first and a second ends of the container, which are the bottom and upper ends of the container in use. The axial flow preferably creates a vertically downward flow from the one or more impellers towards the gas injection system close to the axis of rotation of the first impeller and from the gas injection system vertically upward close to the sidewalls of the container towards the opposite end of the container. More preferably, the step 1010 comprises further generating a radial flow by means of the first impeller. The radial flow preferably creates a flow from the axis of rotation of the one or more impellers towards the container sidewalls. As the flow is above the sparger and near the bottom of the container, the flow interacts with any of the gas bubbles provided by the gas injection system.

Then, the method comprises the optional step 1020 of generating radial flow by the most proximal impeller above the gas injection system. Advantageously, this further enhances that the downward axial flow generated by the axial flow, is transferred radially towards the sidewalls of the liquid container which in turn flows upwards towards the surface of the liquid, creating a circulatory flow into the gas injection system by one or more second impellers above the first impeller. Therefore, in use the container comprises an axial flow vertically flowing from above the one or more second impellers towards the first impeller. Whenever more than one second impellers are used, each second impeller provides an axial flow to the adjacent impellers below itself.

Furthermore, the method comprises the step 1030 of reducing the azimuthal flow generated by one or more of the impellers by at least one baffle axially displaced from the at least one set of impellers and fixed relative to the container at an orientation. The baffle comprises a surface with an orientation at least partially perpendicular to the azimuthal flow generated around any of the axis of rotation of the container.

Finally, the method comprises the step 1040 of injecting the gas into the fluid by means of the gas injection system near the first end of the container.

The injection is preferably done using orifices radially located between the axis of rotation of the impeller closest to the first end of the container and the one or more sidewalls, more preferably homogeneously distributed through the gas injection system surface, wherein the gas injection system is a sparger. Even more preferably, the at least one impeller is a single impeller and the single impeller is shaped to induce at least axial flow of the liquid towards the gas injection system. Optionally, the single impeller is also shaped to induce radial flow from the axis of rotation of the single impeller to the one or more sidewalls.

**Examples**

**Example 1: Study of the effect of the baffles on the azimuthal speed**

Materials and methods

To study the effect of the baffles on the azimuthal speed, a flow simulation was run using virtual stirred tanks. To do so, the ANSYS-Fluent software was used to perform the Computational Fluid Dynamics (CFD). The virtual stirred tanks was defined as follows:

- Reactor vessel with internal height of 1600 mm and internal diameter of 550 mm
- The standard configuration has 3 Rushton with 6 blades per impeller, equally spaced impellers of 175 mm diameter at axial distances from de first end of the agitated tank of 105 mm 505 mm and 905 mm.
- The configuration following the present invention is composed of 4 impeller sets of 275 mm diameter (1 radial impeller as in Fig. 4B, 105 mm from the first end + 3 axial equally spaced impellers as in Fig. 4A, 400 mm distance between impeller centres, 6 blades for the radial impeller, 4 blades per axial impeller with pitch angles 132 of each plane blades of 30 degree.

- Gas generation system is a sparger of 10 mm of pipe diameter. It has with 15 equally spaced orifices of 1 mm at an axial distance to the first end of the bioreactor of 90 mm and a radial distance from the axis of the radial impeller of 150 mm.
- Gas flow rate = 0,8 vvm (reactor volumes per minute).

Three different configurations were studied:

- A stirred tank without baffles.
- A stirred tank with vertical plane baffles attached to the sidewalls.
- A stirred tank with baffles according to the present invention as shown in Fig. 3B with an angle 152 of 30 degrees and a minimum distance to de axis of rotation of 130 mm.

Each model was exposed to the same fluid and gas environments. Particularly, the following parameters were used.

- Fluid: water, viscosity of 0,001 kg m-1 s-1, temperature of 20 °C.
- Gas: air, output flow of 0,8 vvm.

Results

Fig. 7 shows a simulated fluid flow within an agitator tank according to the present invention.

| Type of baffle | Average azimuthal velocity (m/s) |
| --- | --- |
| Without baffles | 0.90 |
| Vertical baffles attached to the sidewalls | 0.79 |
| Baffles according to the present invention | 0.66 |

The results showed that while adding baffles reduces the azimuthal velocity, the baffles according to the present invention further reduce the azimuthal velocity. Therefore, the homogeneity of the bubble distribution in the tank is increased.

**Example 2: Study of the effect of the baffles on the homogeneity**

Materials and methods

To study the effect of the baffles on the homogeneity of the bubbles' distribution, a flow simulation was run using virtual stirred tanks. To do so, the ANSYS-Fluent software was used to perform the CFD. The virtual stirred tanks was defined as in Example 1.
Three different configurations were studied:

- A stirred tank with Rushton impellers and standard vertical baffles, as reference.
- A stirred tank according to present invention with standard vertical baffles.
- A stirred tank and baffles according to present invention.

Each model was exposed to the same fluid and gas environments. Particularly, the following parameters were used.

- Fluid: water, viscosity of 0,001 kg m-1 s-1, temperature of 20 °C.

Gas: air, output flow of 0,8 vvm The stirred tank with Rushton impellers was simulated at a rotational speed of 500 rpm while the other two models were run at 400 rpm. The oxygen transfer constant ($k_{La}$) of each model was registered and the $k_{La}$ of the stirred tank with Rushton impellers was used as a reference.

Results

| Type of agitation | Revolutions of the agitator | Increas e of $k_{La}$ |
|---|---|---|
| Agitator with Rushton impellers and standard vertical baffles | 500 rpm | 0.0% |
| Agitator according to present invention with standard vertical baffles | 400 rpm | 27.9% |
| Agitator and baffles according to present invention | 400 rpm | 39.1% |

The results showed that even at a lower rotation speed, both the impellers according to the present invention and the baffles according to the present invention significantly increase the oxygen transfer constant, up to a 39% when compared to a standard stirred tanks with standard agitator. This shows that the present invention provides a better homogeneity of the bubble's distribution in the stirred tank than a conventional stirred tank.

**Example 3: Comparison of a Computational Fluid Dynamics (CFD) of a stirred tank according to the invention vs a conventional stirred tank**

Materials and methods

To show the advantages of the stirred tank according to the present invention, a flow simulation was run using virtual stirred tanks. To do so, the ANSYS-Fluent software was used to perform the CFD.
The stirred tank A according to the invention was designed as follows:

- Reactor vessel with internal height of 280 mm and internal diameter of 150 mm
- The configuration following the present invention is composed of 2 impeller sets (1 radial impeller with 70 mm diameter as in Fig. 4B, 35 mm from the first end + 1 axial impeller with 70 mm diameter as in Fig. 4A, 105 mm distance between impeller centres, 6 blades for the radial impeller, 4 blades per axial impeller, with pitch angles 132 of each plane blades of 30 degrees.

- Gas generation system is a sparger of 6 mm of pipe diameter. It has with 6 equally spaced orifices of 1 mm at an axial distance to the first end of the bioreactor of 25 mm and a radial distance from the axis of the radial impeller of 25 mm.
- Gas flow rate = 0,8 vvm (reactor volumes per minute).

As a conventional tanks, a stirred tank B with the following characteristics was built

- Reactor vessel with internal height of 280 mm and internal diameter of 150 mm.
- This configuration has 2 Rushton with 6 blades per impeller, equally spaced impellers of 50 mm diameter at axial distances from de first end of the agitated tank of 55 mm and 155 mm.
- The gas generation system and its gas flow rate were identical to that of the stirred tank A.

Both models were exposed to the same fluid and gas environments. Particularly, the following parameters were used.

- Fluid: water, viscosity of 0,001 kg m-1 s-1, temperature of 20 °C.
- Gas: air, output flow was varied from 2 to 8 l/m.
- Agitator rotation speed was varied from 300 to 1000 rpm.

The oxygen transfer constant ($k_{La}$) of both models at different rotational speeds (rpm) and with different gas flow rates measured in litres per minute (lpm) was compared.

Results

As shown in Fig. 8, the reactor A which corresponds to the tank with agitation and aeration system according to the present invention achieved a higher $k_{L_a}$ in all cases than the reactor B which corresponds to the tank with standard agitation and aeration system and it is noteworthy that the tanks A achieves higher $k_{L_a}$ at much lower rotational speeds. The oxygen transfer constant increases when the distribution of the gas bubbles is more uniform, since regions of high bubble concentration (such as those usually near the axis of rotation of impellers) cause bubble coalescence, which reduces the contact surface between gas and liquid. On the other hand, regions of low bubble concentration also have less contact surface between gas and liquid.

**Claims**

1.    An agitator tank for the homogeneous distribution of bubbles in a liquid, comprising:

- a liquid container comprising a first and a second end defining the axial ends of the container and one or more sidewalls extending between the axial ends,
- a gas injection system, located proximal to the first end of the container,
- at least one impeller rotatable about at least one axis of rotation and comprising one or more blades extending to a maximum distance R from the axis of rotation of the corresponding impeller,
- at least one baffle axially displaced from the at least one impeller and fixed relative to the container at an orientation for reducing azimuthal flow generated by the at least one impeller;

wherein one or more impellers of the at least one impeller are shaped and oriented to induce axial flow of the liquid towards the gas injection system and the minimum distance of the at least one baffle to at least one axis of rotation is at most 1.2 R of any of the at least one impeller.

2.    The agitator tank according to claim 1, wherein a gap is provided between the at least one baffle and the one or more sidewalls.

3.    The agitator tank according to any of claims 1 or 2, wherein the at least one impeller is a single impeller and the single impeller is shaped to induce at least axial flow of the liquid towards the gas injection system, optionally wherein the single impeller is also shaped to induce radial flow from the axis of rotation of the single impeller to the one or more sidewalls.

4.    The agitator tank according to claim 3, wherein one or more, and preferably all, of the blades of the single impeller comprise a surface having an average pitch angle between 10° and 60°, preferably between 15° and 50°, wherein the average pitch angle is defined as the average angle formed between the chord line and the plane orthogonal to the axis of rotation of the impeller.

5.    The agitator tank according to any of claims 1 or 2, wherein the at least one impeller comprises a plurality of impellers axially displaced relative to one another, wherein the plurality of impellers comprises:

- a first impeller closest to the gas injection system, the first impeller shaped to induce at least radial flow of the liquid from the axis of rotation of the first impeller to the one or more sidewalls; and
- one or more second impellers farther from the gas injection system, the one or more second impellers shaped to induce at least axial flow of the liquid towards the first impeller.

6.    The agitator tank according to claim 5, wherein one or more, and preferably all, of the blades of the first impeller comprise a surface having an average pitch angle between 50° and 90° and/or wherein one or more, and preferably all, of the blades of the one or more second impellers comprise a surface having and average pitch angle between 10° and 60°, preferably between 15° and 50°; wherein the average pitch angle is defined as the average angle formed between the chord line and the plane orthogonal to the axis of rotation of the impeller on which the blade is located.

7.    The agitator tank according to any of the claims 5 or 6 wherein the minimum distance between two adjoining impellers is less than 3R of any of said impellers.

8. The agitator tank according to any of the claims 5 to 7 wherein the minimum diameter of the second impellers is greater than 0.3 times the square root of the vessel section perpendicular to the axis at the second impeller level, preferably greater than 0.45 times the square root of the vessel section perpendicular to the axis at the second impeller level.

9. The agitator tank according to any of claims 1 to 8, wherein the at least one baffle is folded or curved such that azimuthal flow of the liquid is converted into axial flow.

10. The agitator tank according to any of claims 1 to 9, wherein the minimum distance of the at least one baffle to at least one axis of rotation is less than R of any of the at least one impeller.

11. The agitator tank according to any of claims 1 to 10, wherein the gas injection system comprises injection orifices and wherein the average distance of the injection orifices to the first end is less than the distance R of the first impeller.

12. The agitator tank according to any of claims 1 to 11, wherein the gas injection system is a sparger comprising a plurality of orifices each located radially between the axis of rotation of the impeller closest to the first end of the container and the one or more sidewalls, and wherein the average distance between the orifices of the sparger and the axis of rotation of the impeller closest to the first end of the container is larger than 0,7 R of the impeller closest to the first end of the liquid container.

13. The agitator tank according to any of the claims 1 to 12, wherein the agitator tank comprises more than one impeller when the ratio between the height (H) of the liquid the agitator tank is configured to contain inside the liquid container and the mean of the diameter (<D>) of the one or more sidewalls is greater than 1.5 and more than 2 impellers if said ratio (H/<D>) is greater than 2, wherein the liquid height (H) is the distance of such free liquid surface to the first end the agitated tank, and wherein the mean of the diameter (<D>) is the mean of the square root of the surface of the vessel section perpendicular to the axis along the liquid container.

14. The agitator tank according to any of the claims 1 to 13, wherein the at least one impeller comprises a hub with a radius greater than 0.2 R, preferably greater than 0.3R.

15. A method for homogeneously distributing bubbles in a liquid within an agitator tank, wherein the agitator tank is an agitator tank according to any of the claims 1 to 14 the method comprising the following steps:

   a) generating at least an axial flow towards the first end of the agitator tank by means of one or more impellers of the at least one impeller above the gas injection system, preferably further generating a radial flow by means of the first impeller,
   b) reducing the azimuthal flow generated by one or more of the impellers by the at least one baffle, and
   c) injecting the gas into the fluid by means of the gas injection system proximal to the first end the container.

**Fig. 1**

120

122

Fig. 2A

120

122

Fig. 2B

120

122

Fig. 2C

Fig. 3 A

Fig. 3 B

135

132

**Fig. 4 A**

130

135

135

136

135

3

4

5

**Fig. 4 B**

5

146

142

145

## Fig. 5A

155

## Fig. 5B

Leading edge

Direction of blade rotation

$\alpha(r)$

Relative
flow direction

Chord line

Trailing edge

**Fig. 6**

**Fig. 7**

EP 4 400 206 A1

Fig. 8

Fig. 9

Europäisches
Patentamt

European
Patent Office

Office européen
des brevets

# EUROPEAN SEARCH REPORT

Application Number

EP 23 38 2029

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/355302 A1 (SCHOLZ ALEXANDER [DE] ET AL) 13 December 2018 (2018-12-13) | 1,2,5-15 | INV.<br>B01F23/233 |
| A | * abstract *<br>* figures 1,2 *<br>* paragraphs [0059] – [0069] * | 3,4 | B01F27/86<br>B01F27/902 |
| X | JP S63 104637 A (HITACHI LTD; HITACHI TECHNO ENG) 10 May 1988 (1988-05-10) | 1-4,11, 13-15 | |
| A | * abstract *<br>* figure 1 * | 5-10,12 | |
| X | US 6 250 797 B1 (WEETMAN RONALD J [US]) 26 June 2001 (2001-06-26) | 1,5-8, 11-15 | |
| A | * abstract *<br>* figures 1-10 *<br>* column 5, line 65 – column 6, line 7 * | 2-4,9,10 | |

TECHNICAL FIELDS
SEARCHED      (IPC)

B01F
C12M

The present search report has been drawn up for all claims

| Place of search | Date of completion of the search | Examiner |
|---|---|---|
| The Hague | 3 July 2023 | Krasenbrink, B |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 23 38 2029

This annex lists the patent family members relating to the patent documents cited in the above-mentioned European search report.
The members are as contained in the European Patent Office EDP file on
The European Patent Office is in no way liable for these particulars which are merely given for the purpose of information.

03-07-2023

| Patent document cited in search report | | Publication date | Patent family member(s) | | Publication date |
|---|---|---|---|---|---|
| US 2018355302 | A1 | 13-12-2018 | AU 2016261296 | A1 | 07-12-2017 |
| | | | BR 112017024104 | A2 | 31-07-2018 |
| | | | CA 2983077 | A1 | 17-11-2016 |
| | | | CN 107636140 | A | 26-01-2018 |
| | | | EA 201792456 | A1 | 31-07-2018 |
| | | | EP 3093336 | A1 | 16-11-2016 |
| | | | EP 3294859 | A1 | 21-03-2018 |
| | | | KR 20180004145 | A | 10-01-2018 |
| | | | US 2018355302 | A1 | 13-12-2018 |
| | | | WO 2016180823 | A1 | 17-11-2016 |
| | | | ZA 201708219 | B | 29-05-2019 |
| JP S63104637 | A | 10-05-1988 | JP H0543405 | B2 | 01-07-1993 |
| | | | JP S63104637 | A | 10-05-1988 |
| US 6250797 | B1 | 26-06-2001 | AU 6051799 | A | 26-04-2000 |
| | | | CA 2345981 | A1 | 13-04-2000 |
| | | | EP 1124627 | A1 | 22-08-2001 |
| | | | US 6250797 | B1 | 26-06-2001 |
| | | | WO 0020109 | A1 | 13-04-2000 |
| | | | ZA 200103405 | B | 11-12-2001 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2019168177 A1
- KR 1020160039907 A
- US 2019078045 A1
- WO 2019126660 A1